# EUROPÄISCHE PATENTANMELDUNG

(11) **EP 0 941 720 A2**
(43) Veröffentlichungstag der Anmeldung: **15.09.1999**
(21) Anmeldenummer: 98124726.5
(22) Anmeldetag: 28.12.1998
(51) Int. Cl.: A61F 2/68, A61F 2/64

(54) **Prothesenbremsgelenk**

(30) Priorität: 11.03.1998 DE 19810385
(71) Anmelder: Otto Bock Orthopädische Industrie Besitz- und Verwaltungs-Kommanditgesellschaft, 37115 Duderstadt (DE)
(72) Erfinder: Wagner, Helmut, 37115 Duderstadt (DE); Krukenberg, Manfred, 37115 Duderstadt (DE)
(74) Vertreter: Gramm, Werner, Prof. Dipl.-Ing.

(57) **Zusammenfassung**

Die Erfindung betrifft ein Prothesenbremsgelenk, insbesondere Bremskniegelenk für eine Beinprothese, mit einem Gelenkoberteil, einem Gelenkunterteil und einer diese beiden Gelenkteile verschwenkbar miteinander verbindenden, drehfest mit dem Gelenkunterteil verbundenen, als Bremsachse ausgebildeten Gelenkachse, die von einer sich auf ihr abstützenden Bremseinrichtung umschlossen ist, die in einer zylindrischen, zusammen mit der Gelenkachse eine Ringkammer bildenden Ausnehmung in einem ein Gelenkmittelteil bildenden Klemmteil angeordnet ist, an dem das Gelenkoberteil über eine Schwingachse derart gelenkig gelagert ist, daß das Gelenkoberteil bei Belastung das Klemmteil und dadurch die Bremseinrichtung beaufschlagt und so eine Bremswirkung auf die Gelenkachse ausübt. Zur Verbesserung der Bremseinrichtung wird erfindungsgemäß vorgeschlagen, daß die Bremseinrichtung durch parallelachsig zu der in äußeren Achslagern gelagerten Gelenkachse angeordnete, die Gelenkachse voll umfänglich umgebende Nadelrollen gebildet ist, die einen inneren, sich auf der Gelenkachse abstützenden Nadelrollenring und einen äußeren, sich an der äußeren Mantelfläche der Ringkammer abstützenden Nadelrollenring umfassen, dessen Nadelrollen zahnartig zwischen die inneren Nadelrollen eingreifen, und durch einen am Gelenkoberteil sitzenden Schaltkeil, der bei Belastung des Gelenkoberteils in - bezogen auf die Gelenkachse - angenähert radialer Wirkrichtung zwei benachbarte innere Nadelrollen in Umfangsrichtung der Gelenkachse auseinanderdrückt.

## Beschreibung

Die Erfindung betrifft ein Prothesenbremsgelenk, insbesondere Bremskniegelenk für eine Beinprothese, mit einem Gelenkoberteil, einem Gelenkunterteil und einer diese beiden Gelenkteile verschwenkbar miteinander verbindenden, drehfest mit dem Gelenkunterteil verbundenen, als Bremsachse ausgebildeten Gelenkachse, die von einer sich auf ihr abstützenden Bremseinrichtung umschlossen ist, die in einer zylindrischen, zusammen mit der Gelenkachse eine Ringkammer bildenden Ausnehmung in einem ein Gelenkmittelteil bildenden Klemmteil angeordnet ist, an dem das Gelenkoberteil über eine Schwingachse derart gelenkig gelagert ist, daß das Gelenkoberteil bei Belastung das Klemmteil und dadurch die Bremseinrichtung beaufschlagt und so eine Bremswirkung auf die Gelenkachse ausübt. Dabei können in kinematischer Hinsicht Gelenkober- und Gelenkunterteil miteinander vertauscht werden, wobei dann die Gelenkachse drehfest mit dem Gelenkoberteil verbunden wäre.

Eine derartige Ausführungsform läßt sich der DE 195 11 890 C1 entnehmen. Die Bremseinrichtung besteht hier aus einer auf der Gelenkachse als Lager laufenden Bremsbuchse, die drehfest mit dem Klemmteil verbunden ist. Die Bremsbuchse ist von einer kreisringförmigen Bremskammer umschlossen, die mit einem inkompressiblen Medium gefüllt ist und mit einem im Klemmteil integrierten, geschlossenen Hohlraum in flüssigkeitsaustauschender Verbindung steht, in den ein das inkompressible Medium beaufschlagender Druckkolben ragt, an dem sich das Gelenkoberteil abstützt.

Der Erfindung liegt die Aufgabe zugrunde, für das eingangs beschriebene Prothesenbremsgelenk eine verschleißarme, schnell ansprechende und eine hohe Bremskraft ausübende Bremseinrichtung zu entwickeln.

Diese Aufgabe wird erfindungsgemäß dadurch gelöst, daß die Bremseinrichtung durch parallelachsig zu der in äußeren Achslagern gelagerten Gelenkachse angeordnete, die Gelenkachse voll umfänglich umgebende Nadelrollen gebildet ist, die einen inneren, sich auf der Gelenkachse abstützenden Nadelrollenring und einen äußeren, sich an der äußeren Mantelfläche der Ringkammer abstützenden Nadelrollenring umfassen, dessen Nadelrollen zahnartig zwischen die inneren Nadelrollen eingreifen, und durch einen am Gelenkoberteil sitzenden Schaltkeil, der bei Belastung des Gelenkoberteils in - bezogen auf die Gelenkachse - angenähert radialer Wirkrichtung zwei benachbarte innere Nadelrollen in Umfangsrichtung der Gelenkachse auseinanderdrückt.

In einem ersten Ausführungsbeispiel ist es zweckmäßig, wenn die mit jeweils einem lichten Umfangsabstand voneinander angeordneten inneren Nadelrollen gleichen Durchmesser aufweisen. Dabei kann der Durchmesser der äußeren Nadelrollen dem der inneren Nadelrollen entsprechen. Der Schaltkeil stützt sich vorzugsweise formschlüssig auf einer äußeren Nadelrolle ab.

Um sicherzustellen, daß sich die Nadelrollen des inneren Nadelrollenringes nicht berühren (was bei einem Achsversatz möglich wäre), ist es zweckmäßig, wenn jeweils die inneren sowie die äußeren Nadelrollen stirnseitig mit Spiel zwischen Kugellagerringen radial geführt sind.

Für eine abgewandelte Ausführungsform ist es vorteilhaft, wenn sich der innere Nadelrollenring aus Nadelrollen mit abwechselnd kleinerem und größerem Durchmesser zusammensetzt, wobei die Nadelrollen größeren Durchmessers als Abstandshalter zwischen den Nadelrollen kleineren Durchmessers fungieren und an der äußeren Mantelfläche der Ringkammern anliegen, während der äußere Nadelring ausschließlich Nadelrollen kleineren Durchmessers umfaßt, die jeweils zwischen eine Nadelrolle größeren Durchmessers und eine dieser in Beugerichtung folgende Nadelrolle kleineren Durchmessers des inneren Nadelrollenringes eingreifen.

Um ein schnelles Ansprechen der Bremseinrichtung zu gewährleisten, ist es vorteilhaft, wenn die radiale Höhe der Ringkammer nur wenige Zehntelmillimeter kleiner ist als die Summe der Durchmesser der beiden übereinanderliegenden Nadelrollen kleineren Durchmessers.

Weitere Merkmale der Erfindung sind Gegenstand der Unteransprüche und werden in Verbindung mit weiteren Vorteilen der Erfindung anhand zweier Ausführungsbeispiele näher erläutert.

In der Zeichnung sind zwei als Beispiele dienende Ausführungsformen der Erfindung dargestellt. Es zeigen:
- Figur 1 -: in Seitenansicht ein Bremskniegelenk in gestreckter Lage;
- Figur 2 -: einen lotrechten Schnitt gemäß der Linie II-II in Figur 1;
- Figur 3 -: einen lotrechten Schnitt gemäß der Linie III-III in Figur 2;
- Figur 4 -: das Bremskniegelenk gemäß Figur 1 in gebeugter Stellung;
- Figur 5 -: einen Schnitt gemäß Figur 3 durch das gebeugte Bremskniegelenk gemäß Figur 4;
- Figur 6 -: in einer Darstellung gemäß Figur 2 einen Schnitt durch eine abgewandelte Ausführungsform;
- Figur 7 -: das Bremskniegelenk gemäß Figur 6 in einer Schnittdarstellung gemäß Figur 3 und
- Figur 8 -: das Bremskniegelenk gemäß Figur 7 in gebeugter Stellung.

Die Figuren zeigen ein Bremskniegelenk für eine Beinprothese. Dieses Bremskniegelenk besteht im wesentlichen aus einem Gelenkoberteil 1, einem Gelenkunterteil 2 und einer diese beiden Gelenkteile 1, 2 verschwenkbar miteinander verbindenden, drehfest mit dem Gelenkunterteil 2 verbundenen und als Bremsachse ausgebildeten Gelenkachse 3, die in äußeren Achslagern 4 gelagert ist. Ein ein Gelenkmittelteil bildendes Klemmteil 5 umschließt mit einer zylindrischen Ausnehmung konzentrisch die Gelenkachse 3 und bildet mit dieser eine Ringkammer 6, in der eine Bremseinrichtung angeordnet ist. Das Gelenkoberteil 1 ist an dem Klemmteil 5 über eine Schwingachse 7 gelenkig gelagert.

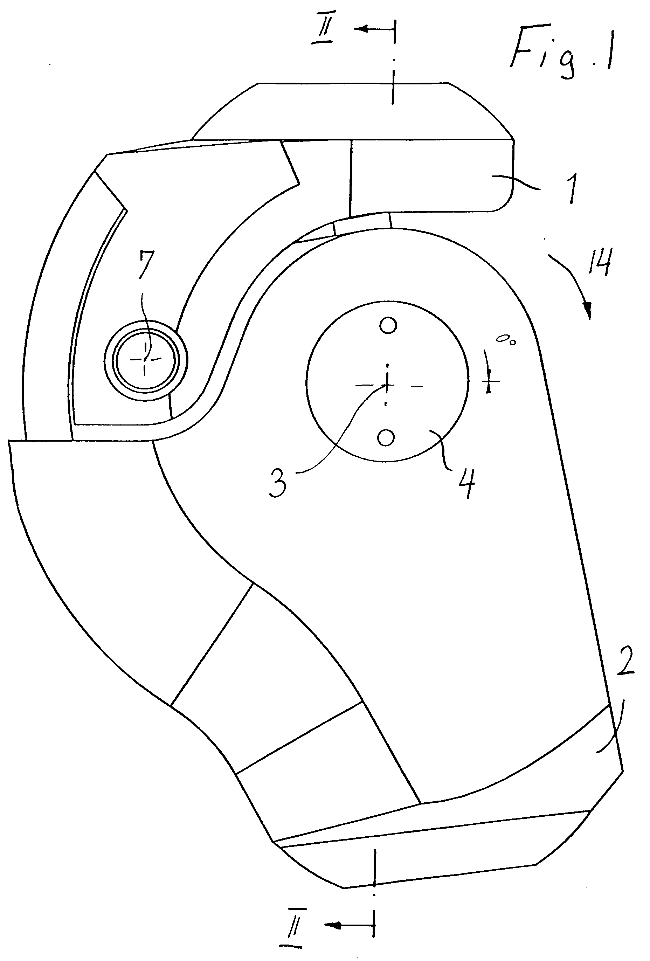

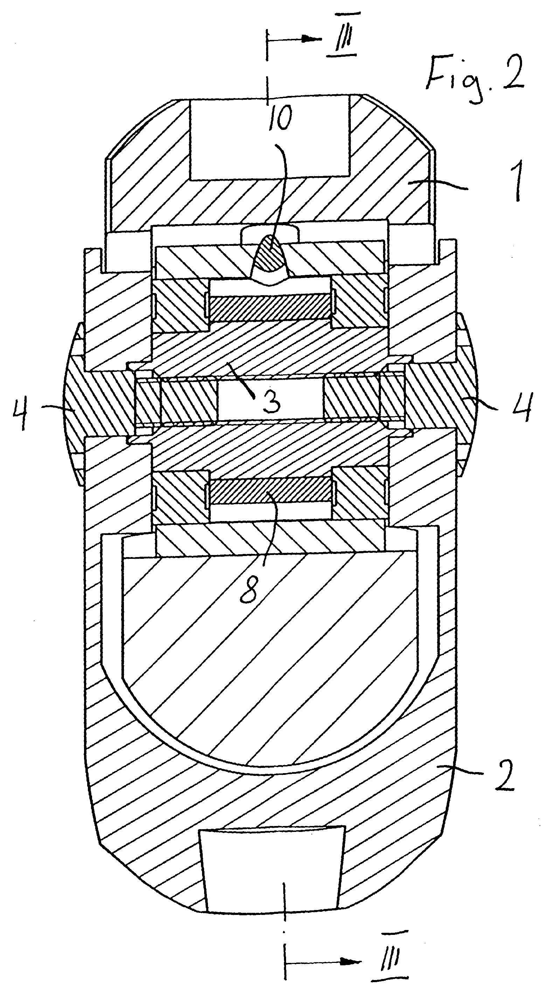

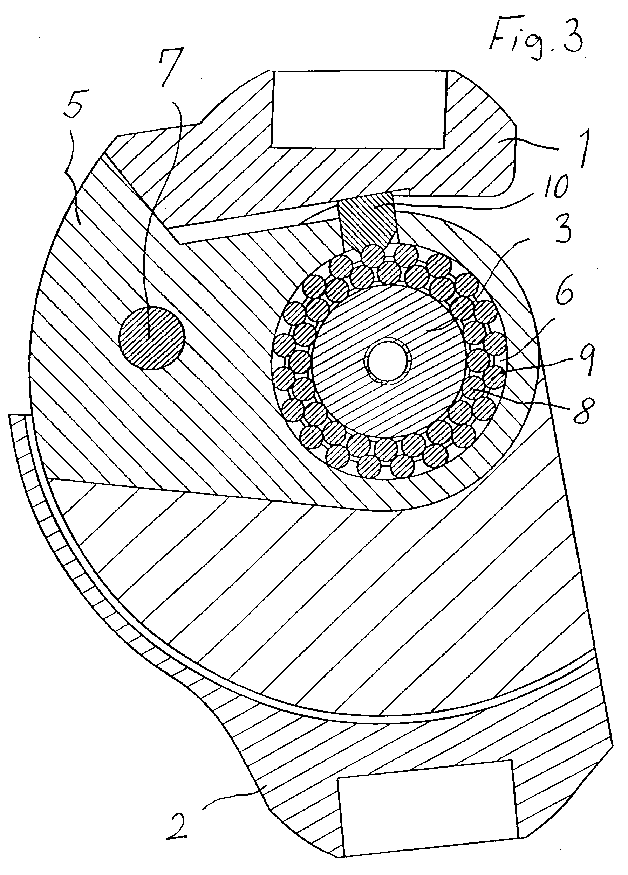

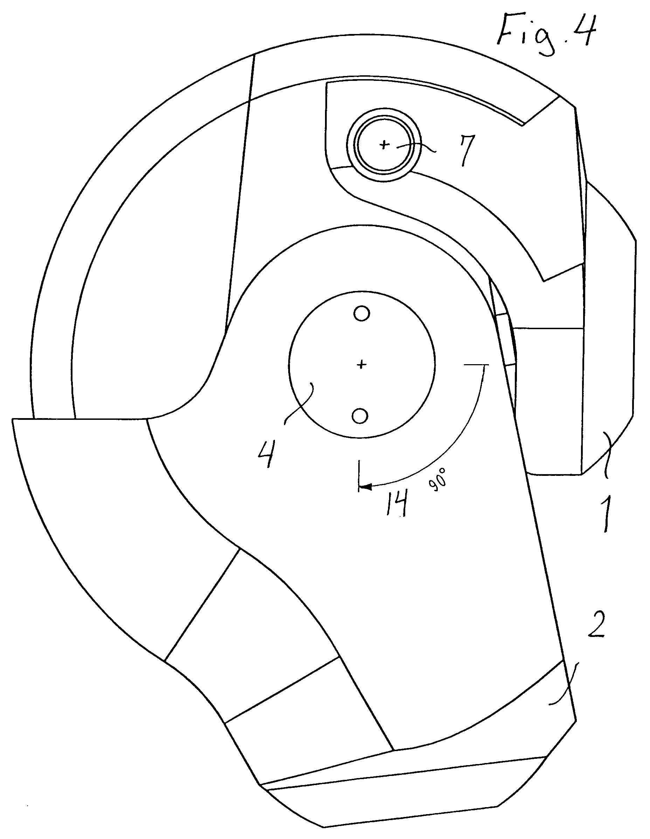

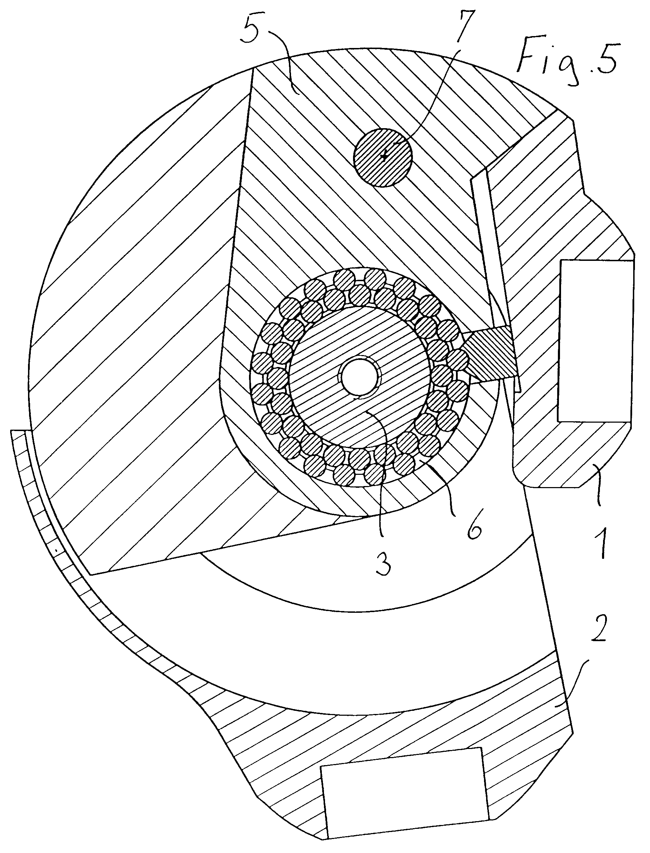

Bei dem Ausführungsbeispiel gemäß den Figuren 1 bis 5 ist die genannte Bremseinrichtung gebildet durch parallelachsig zu der Gelenkachse 3 angeordnete und diese voll umfänglich umgebende Nadelrollen 8, 9, die einen inneren, sich auf der Gelenkachse 3 abstützenden Nadelrollenring und einen äußeren, sich an der äußeren Mantelfläche der Ringkammer 6 abstützenden Nadelrollenring umfassen, dessen Nadelrollen 9 zahnartig zwischen die inneren Nadelrollen 8 eingreifen. Die inneren Nadelrollen 8 weisen jeweils gleichen Durchmesser auf und sind mit jeweils einem lichten Umfangsabstand voneinander angeordnet. Der Durchmesser der äußeren Nadelrollen 9 entspricht dem der inneren Nadelrollen 8. Die inneren Nadelrollen 8 sind ebenso wie die äußeren Nadelrollen 9 stirnseitig mit Spiel zwischen nicht näher dargestellten Kugellagerringen radial geführt.

Am Gelenkoberteil 1 sitzt ein nach unten ragender Schaltkeil 10, der sich formschlüssig auf einer äußeren Nadelrolle 9 abstützt und bei Belastung des Gelenkoberteils 1 in - bezogen auf die Gelenkachse 3 - angenähert radialer Wirkrichtung zwei benachbarte innere Nadelrollen 8 in Umfangsrichtung der Gelenkachse 3 auseinanderdrückt. Die dadurch hervorgerufene Verkeilung zwischen den inneren und äußeren Nadelrollen 8, 9 führt zu einer Klemmverbindung zwischen dem Gelenkoberteil 1 und der Gelenkachse 3 und damit zu einer Blockierung der Beugung.

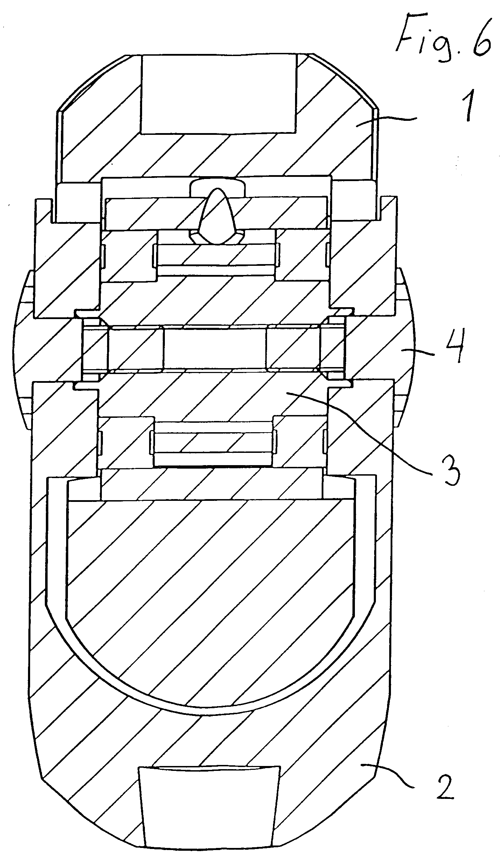

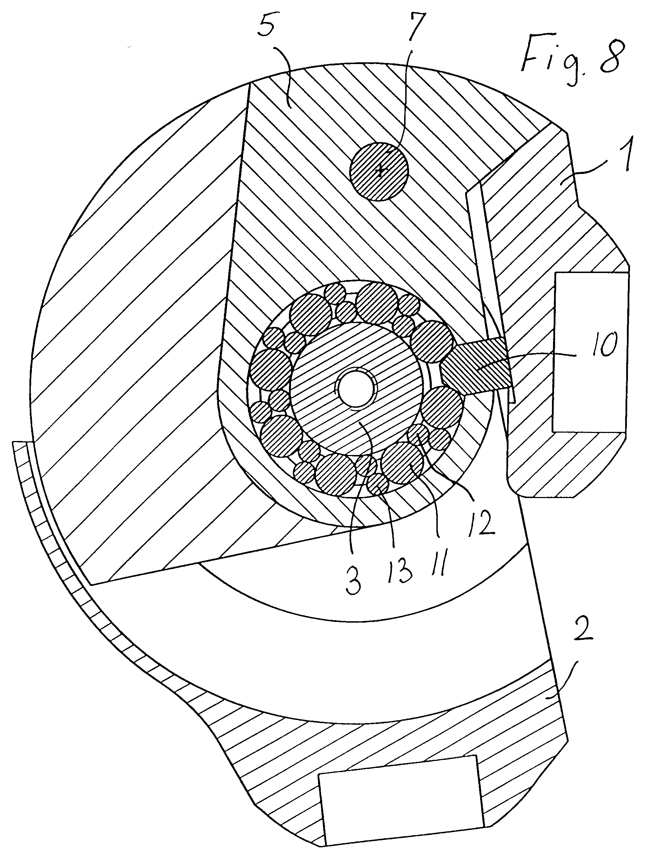

Bei dem zweiten Ausführungsbeispiel gemäß den Figuren 6 bis 8 setzt sich der innere Nadelrollenring aus Nadelrollen mit abwechselnd kleinerem und größerem Durchmesser zusammen, wobei die Nadelrollen 11 größeren Durchmessers als Abstandshalter zwischen den Nadelrollen 12 kleineren Durchmessers fungieren und an der äußeren Mantelfläche der Ringkammer 6 anliegen. Der äußere Nadelring umfaßt ausschließlich Nadelrollen 13 kleineren Durchmessers, die jeweils zwischen eine Nadelrolle 11 größeren Durchmessers und eine dieser in Beugerichtung 14 folgende Nadelrolle 12 kleineren Durchmessers des inneren Nadelrollenringes eingreifen. Dabei weisen in dem dargestellten Ausführungsbeispiel die Nadelrollen 12 und 13 kleineren Durchmessers jeweils gleichen Durchmesser auf.

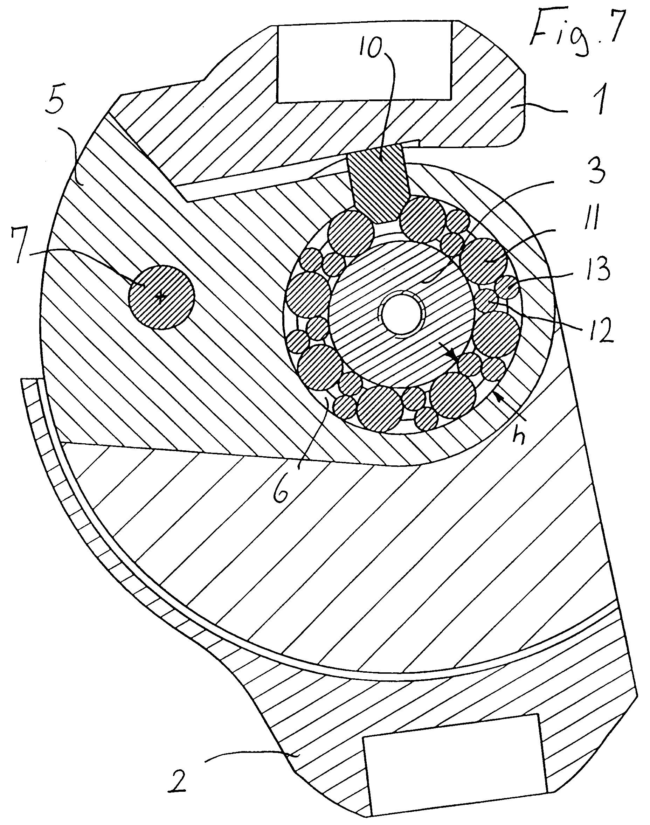

Die radiale Höhe h der Ringkammer 6 ist nur wenige Zehntelmillimeter kleiner als die Summe der Durchmesser der beiden übereinanderliegenden Nadelrollen 12, 13 kleineren Durchmessers. Beträgt z. B. der Durchmesser der Nadelrollen 12, 13 3 mm, dann beträgt die Spalthöhe h der Ringkammer 6 vorzugsweise 5, 8 mm. Figur 7 läßt erkennen, daß die Rollenpaare 12, 13 einen der Beugerichtung 14 entgegengerichteten Anstellwinkel aufweisen.

Bei dem zweiten Ausführungsbeispiel stützt sich der Schaltkeil 10 mit einer Keilschräge auf zwei benachbarten Nadelrollen 11 größeren Durchmessers ab und beaufschlagt sie dadurch - einander entgegengesetzt - in Umfangsrichtung der Ringkammer 6.

Bei beiden Ausführungsbeispielen kann die Mantelfläche der Gelenkachse 3 und/oder der Ringkammer 6 gehärtet sein.

Bei beiden Ausführungsformen führen die Nadelrollen zu einer exakten Justierung der Gelenkachse 3. Bei dem ersten Ausführungsbeispiel ist für das Ansprechen der Bremseinrichtung ein etwas höherer Druck über den Schaltkeil 10 erforderlich, während die Bremseinrichtung des zweiten Ausführungsbeispieles schon bei geringeren Belastungsdrücken anspricht. Bei dem ersten Ausführungsbeispiel führt eine Belastung des Gelenkoberteils 1 zu einer Blockierung des Gelenkoberteils 1 gegenüber der Gelenkachse 3 und zwar sowohl in Beugerichtung 14 als auch in Streckrichtung, während bei dem zweiten Ausführungsbeispiel die Blockierung nur in Beugerichtung 14 wirksam wird.

## Patentansprüche

1. Prothesenbremsgelenk, insbesondere Bremskniegelenk für eine Beinprothese, mit einem Gelenkoberteil (1), einem Gelenkunterteil (2) und einer diese beiden Gelenkteile (1, 2) verschwenkbar miteinander verbindenden, drehfest mit dem Gelenkunterteil (2) verbundenen, als Bremsachse ausgebildeten Gelenkachse (3), die von einer sich auf ihr abstützenden Bremseinrichtung umschlossen ist, die in einer zylindrischen, zusammen mit der Gelenkachse (3) eine Ringkammer (6) bildenden Ausnehmung in einem ein Gelenkmittelteil bildenden Klemmteil (5) angeordnet ist, an dem das Gelenkoberteil (1) über eine Schwingachse (7) derart gelenkig gelagert ist, daß das Gelenkoberteil (1) bei Belastung das Klemmteil (5) und dadurch die Bremseinrichtung beaufschlagt und so eine Bremswirkung auf die Gelenkachse (3) ausübt, **dadurch gekennzeichnet, daß** die Bremseinrichtung durch parallelachsig zu der in äußeren Achslagern (4) gelagerten Gelenkachse (3) angeordnete, die Gelenkachse (3) voll umfänglich umgebende Nadelrollen (8, 9; 11, 12, 13) gebildet ist, die einen inneren, sich auf der Gelenkachse (3) abstützenden Nadelrollenring und einen äußeren, sich an der äußeren Mantelfläche der Ringkammer (6) abstützenden Nadelrollenring umfassen, dessen Nadelrollen (9; 13) zahnartig zwischen die inneren Nadelrollen (8; 12) eingreifen, und durch einen am Gelenkoberteil (1) sitzenden Schaltkeil (10), der bei Belastung des Gelenkoberteils (1) in - bezogen auf die Gelenkachse (3) - angenähert radialer Wirkrichtung zwei benachbarte innere Nadelrollen (8; 11) in Umfangsrichtung der Gelenkachse (3) auseinanderdrückt.

2. Prothesenbremsgelenk nach Anspruch 1, **dadurch gekennzeichnet, daß** die mit jeweils einem lichten Umfangsabstand voneinander angeordneten inneren Nadelrollen (8) gleichen Durchmesser aufweisen. (Figur 3)

3. Prothesenbremsgelenk nach Anspruch 2, **dadurch gekennzeichnet, daß** der Durchmesser der äußeren Nadelrollen (9) dem der inneren Nadelrollen (8) entspricht.

4. Prothesenbremsgelenk nach Anspruch 2 oder 3, **dadurch gekennzeichnet, daß** sich der Schaltkeil (10) formschlüssig auf einer äußeren Nadelrolle (9) abstützt.

5. Prothesenbremsgelenk nach Anspruch 2, 3 oder 4, **dadurch gekennzeichnet, daß** jeweils die inneren sowie die äußeren Nadelrollen (8, 9) stirnseitig mit Spiel zwischen Kugellagerringen radial geführt sind.

6. Prothesenbremsgelenk nach Anspruch 1, **dadurch gekennzeichnet, daß** sich der innere Nadelrollenring aus Nadelrollen (11, 12) mit abwechselnd kleinerem und größerem Durchmesser zusammensetzt, wobei die Nadelrollen (11) größeren Durchmessers als Abstandshalter zwischen den Nadelrollen (12) kleineren Durchmessers fungieren und an der äußeren Mantelfläche der Ringkammern (6) anliegen, während der äußere Nadelring ausschließlich Nadelrollen (13) kleineren Durchmessers umfaßt, die jeweils zwischen eine Nadelrolle (11) größeren Durchmessers und eine dieser in Beugerichtung (13) folgende Nadelrole (12) kleineren Durchmessers des inneren Nadelrollenringes eingreifen. (Figur 7)

7. Prothesenbremsgelenk nach Anspruch 6, **dadurch gekennzeichnet, daß** die radiale Höhe (h) der Ringkammer (6) nur wenige Zehntelmillimeter kleiner ist als die Summe der Durchmesser der beiden übereinanderliegenden Nadelrollen (12, 13) kleineren Durchmessers.

8. Prothesenbremsgelenk nach Anspruch 6 oder 7, **dadurch gekennzeichnet, daß** sich der Schaltkeil (10) mit einer Keilschräge auf zwei benachbarten Nadelrollen (11) größeren Durchmessers abstützt.

9. Prothesenbremsgelenk nach einem der vorhergehenden Ansprüche, **dadurch gekennzeichnet, daß** die Mantelflächen der Gelenkachse (3) und/oder der Ringkammer (6) gehärtet ist bzw. sind.
